# EUROPEAN PATENT SPECIFICATION

(11) **EP 2 838 511 B1**
(45) Date of publication and mention of the grant of the patent: **30.05.2018**
(21) Application number: 13778272.8
(22) Date of filing: 16.04.2013
(51) Int. Cl.: A61K 9/10, A61K 9/70, A61K 9/06, A61K 38/28, A61K 38/18, A61P 27/02, A61K 9/00, A61K 45/06, A61K 38/27, A61K 31/573

(54) **OCULAR DRUG DELIVERY SYSTEM**
OKULARES WIRKSTOFFFREISETZUNGSSYSTEM
SYSTÈME D'ADMINISTRATION DE MÉDICAMENT OCULAIRE

(30) Priority: 16.04.2012 US 201261624730 P; 15.03.2013 US 201313835729
(43) Date of publication of application: 25.02.2015
(73) Proprietor: Jade Therapeutics, LLC, Park City, UT 84098 (US)
(72) Inventor: WIROSTKO, Barbara, Park City, UT 84098 (US)
(74) Representative: Walker, Ross Thomson
(86) International application number: PCT/US2013/036807
(87) International publication number: WO 2013/158661

(56) References cited:
- WO-A2-2012/092510
- US-A1- 2005 085 758
- US-A1- 2008 113 027
- US-A1- 2010 209 384
- US-A1- 2010 330 143
- US-B2- 7 858 582
- GUANGHUI YANG ET AL: "Thiolated Carboxymethyl-Hyaluronic-Acid-Based Biomaterials Enhance Wound Healing in Rats, Dogs, and Horses", ISRN VETERINARY SCIENCE, vol. 6, no. 5, 11 January 2012 (2012-01-11), pages 442-7, XP55225542, ISSN: 2090-4452, DOI: 10.1111/j.1463-5224.2010.00771.x
- JEONG-A YANG ET AL: "Molecular design of hyaluronic acid hydrogel networks for long-term controlled delivery of human growth hormone", SOFT MATTER, vol. 7, no. 3, 7 April 2011 (2011-04-07), page 868, XP55225552, GB ISSN: 1744-683X, DOI: 10.1039/c0sm01011a
- PARENTIN, F. ET AL.: 'Central corneal thickness in children with growth hormone deficiency' ACTA OPHTHALMOLOGICA vol. 88, 2010, pages 692 - 694, XP055117031
- SANDERS, E. J. ET AL.: 'Endogenous growth hormone in human retinal ganglion cells correlates with cell survival' MOLECULAR VISION vol. 15, 2009, pages 920 - 926, XP055117033

## Description

### BACKGROUND

Persistent corneal epithelial defects (PCED) in neurotrophic corneas can be defined as a loss of the integrity of the corneal surface and/or a defect in the epithelium, whether caused by injury or disease, which persists for weeks, months, or even years. The epithelial defects are due to an underlying abnormality in the corneal innervations, lack of innervations, injury to the corneal nerves, inflammation due to the defects, and abnormal tear production. Corneal stromal ulceration may or may not be associated with these neurotrophic corneas from the resulting epithelial defects. Underlying disease states that may result in such defects include: surgical intervention such as refractive surgery and or corneal transplant; previous herpes simplex or herpes zoster infection; neurotrophic keratitis after damage to or loss of the fifth cranial nerve function which can be associated with disease states such as diabetes; exposure keratitis secondary to a Bell's Palsy and mucin-deficient dry eye states, e.g. occurring after chemical, foreign body, and burn injuries, in patients with lid apposition abnormalities and dysfunctional tear film. Non-healing corneal epithelial defects may also occur after ocular surgery or other physical injuries to the cornea even with chronic and overnight contact lens wear. Refractive surgery is a common cause of corneal innervation abnormalities and very often leads to dry eye and various degrees of corneal defects. If corneal innervations are not improved with successful post surgery treatments, chronic dry eye can be a very persistent and disabling problem with chronic pain, ocular discomfort, corneal defects, and blurred vision. If corneal defects persist with the dry eye; corneal ulcers, corneal scarring and opacification may result in subsequent vision loss.

Corneal wound healing, corneal re-epithelization, and corneal re-innervation is a highly regulated process that involves the reorganization, migration, and proliferation of epithelial cells and neural cells from the limbal into the corneal epithelium and corneal stroma. Rapid re-innervation of the injured area is important in restoring normal corneal sensation, reflex lacrimal tear production and hence normal basal tear production which will restore the ocular surface and protect the cornea and establish a normal and healthy environment, such that if the cornea does have epithelial defects, it can now stimulate and 'sense" a normal healing environment and properly activate the lacrimal gland to secrete tears to protect the surface.

Document US 7 858 582 discloses a gel formulation comprising sodium hyaluronate as a matrix polymer and rHGH. The gel improves corneal cicatrization.

### SUMMARY

The present technology includes systems that can be used in creating a healing therapy for ocular pathology and corneal disease where there is an underlying defect in the innervations to the cornea and the ocular surface such that decreased corneal sensation has resulted in corneal epithelial defects, dry eye, exposure keratopathy and/or other related ocular surface diseases. The composition is configured for placement in, around, or on an eye of the subject, and the composition provides controlled release of an amount of the active agent to the eye effective to promote ocular surface and corneal neural regeneration. The formulation includes an active agent that is human growth hormone (HGH), recombinant human growth hormone (rHGH), or an HGH mimic. The composition can be formulated as a topical solution or gel, emulsion, ointment, insert and or film etc. formulation that can be inserted, applied topically, sprayed and/or injected. The composition is configured for placement in the eye of a subject, and provides controlled release of an effective amount of the active agent to the eye. The composition can be configured as a daily topical formulation, sustained topical formulation, injections, spray, gel, ointment, depot, film or the like.

In accordance with the present technology, the composition can be formed of a microparticle suspension, a nanoparticle suspension, a monolithic rod, film, or a gel. The composition can be shaped for subconjunctival, subtenons, cul de sac, conjunctiva, or on the cornea, limbus, intra corneal, periocular region, sub-Tenon's space, or sub scleral placement, and/or peribulbar or retrobulbar deposit. In another embodiment, the composition can be a depot and or film placed under or within the ocular. Further, the composition can be injectable or insertable. This polymer matrix can be delivered directly to the target tissue or placed in a suitable delivery device which is either biodegradable or can be removed upon completion of the drug delivery.

The composition can provide controlled release of the active agent for a day and /or for an extended duration, e.g. from several hours to about 200 days. Release of the active agent can further exhibit zero-order kinetics for substantially the entire release duration with a tapering off as the drug substantially completes release. The controlled release can also exhibit near zero order kinetics for substantially the entire release duration and can optionally be delivered with and or without a burst. Release modes provided include continuous release and pulsed release. The amount of active agent released by the depot can be a bolus with and without an up to zero-order kinetics for substantially the entire duration. In another aspect, the concentration of the active agent in the matrix is from about 0.05 µg to about 100 µg per milliliter.

The polymer matrix of the delivery composition can include a bioerodible polymer that erodes to provide a rate of controlled release. The polymer matrix according to the invention is a bioerodible polymer comprising a cross- linked hyaluronic acid of a thiolated carboxymethyl hyaluronic acid cross-linked with poly(ethylene glycol) diacrylate. The formulation can be dispersed in the polymer matrix as a solid, a powder, a gel, or an emulsion. The formulation can further include a second bioactive agent, such as, but not limited to, antibiotics, anti-microbial agents (e.g. anti-virals, anti-fungals, etc.) anti-inflammatory steroids, non-steroidal anti-inflammatory drugs, analgesics, artificial tears solutions, cellular adhesion promoters, growth factors, decongestants, anticholinesterases, glaucoma hypotensive agents, anti angiogenesis drugs (e.g. anti VEGFs), antiallergenics, or combinations of any of these. In a particular embodiment, the depot is situated adjacent to a rate controlling diffusion barrier.

A method of making an ocular drug delivery depot, including compositions for the system described above, includes dispersing a formulation including an active agent in a polymer matrix selected to provide controlled release of an amount of the active agent to the eye. The drug delivery composition according to the invention is for use in promoting healing of corneal innervations and secondary corneal wounds in a subject, wherein a drug delivery composition is placed in an eye of the subject. The drug delivery composition includes a formulation including an active agent dispersed in a polymer matrix that provides continuous controlled release of an effective amount of the active agent to the eye. In a particular embodiment, placement can be made subconjunctivally, more particularly in subconjunctival locations such as the limbus, the periocular region, sub-Tenon's space, sub sclera, sub corneal and the retrobulbar space. In a more particular example, placement of the composition is deliverable by injection. In another embodiment, the composition is placed under or within a contact lens (e.g. collagen or other dissolvable matrix, or absorbable suture material (Poly lactic glycolic acid (PLGA) and poly lactic acid (PLA)) or other silicone based matrix). In still another embodiment a signal can be applied to the drug delivery system after implantation to alter the controlled release. The signal may be a remote signal. In a particular example, the controlled release occurs via iontophoresis.

### BRIEF DESCRIPTION OF THE DRAWINGS

For a further understanding of the nature and advantage of the present disclosure, reference is being made to the following detailed description of embodiments and in connection with the accompanying drawings, in which:
FIG. 1 shows data results obtained from the study of Example 1 in accordance with one embodiment of the present disclosure; and
FIG. 2 shows data results obtained from the study of Example 3 in accordance with another embodiment of the present disclosure.

### DETAILED DESCRIPTION

In describing embodiments of the present invention, the following terminology will be used.

The singular forms "a," "an," and "the" include plural referents unless the context clearly dictates otherwise. Thus, for example, reference to "an active agent" includes reference to one or more of such pellets and "dispersing" includes one or more of such steps.

As used herein, the term "subject" refers to a mammal. Non-limiting examples of mammals can include rats, mice, dogs, cats, rabbits, horses, non-human primates, and humans. In one preferred aspect, the subject is a human.

As used herein, the term "substantially" refers to the complete or nearly complete extent or degree of an action, characteristic, property, state, structure, item, or result. For example, an object that is "substantially" enclosed would mean that the object is either completely enclosed or nearly completely enclosed.

Compounds that can accelerate neuronal innervations in the cornea can also promote corneal wound healing by increasing the migration and proliferation of cornea and conjunctival epithelial cells. Such a compound can be of interest because of their major potential benefit for patients with epithelial damage such as from dry eye, surgical and non-surgical trauma, refractive interventions, corneal abrasion, non healing corneal ulcers and neurotrophic corneas secondary to diabetes, trauma, surgery, cranial nerve palsies, and herpetic keratitis. Patients suffering from corneal de-innervation can benefit from pharmaco-therapeutic agents that enhance the healing of the cornea through stimulating neuronal growth factor receptors, epidermal growth factor, and/or insulin growth factors receptors and or other receptors such as human growth factor receptors that would allow for improved corneal health, improved tear secretion and an improved ocular surface environment with elevated levels of goblet mucin, aqueous, and meibomian gland lipid production.

Accordingly, in one aspect an ocular drug delivery system is provided comprising a composition having formulation including an active agent that is HGH, rHGH, an rHGH mimic, or a combination thereof, where the formulation is dispersed in a pharmaceutical carrier. Furthermore, the composition is configured for placement in or on an eye of the subject, and can provide controlled release of an amount of the active agent to the eye effective to promote ocular surface and corneal neural regeneration.

HGH is a hydrophilic protein with a molecular weight of 22Kda composed of 191 amino acids. HGH is a member of the somatotropin/prolactin family of hormones, is produced from the pituitary gland, and is required for normal human growth and development. HGH modifies a variety of physiological functions by stimulating the expression of proteins such as, but not limited to, insulin-like growth factor I, neuronal growth factor, and epidermal growth factor. HGH increases muscle mass, promotes lipolysis, augments wound healing, and reduces liver uptake of glucose. There is also clinical experience of the use of HGH in adults and children for insufficient growth related abnormalities. It is noted that the term "HGH" as used throughout the present disclosure can refer to, and be used interchangeably with, HGH, rHGH, and HGH mimics, unless the context clearly indicates otherwise.

Without wishing to be bound by theory, it is believed that dermal and corneal wound healing by HGH up-regulates and cross-talks with IGF. Thus, it is believed that HGH is activating IGF and altering IGFBP in the ocular tears and ocular tissue (corneal and conjunctiva). By activating IGF and increasing IGF, corneal limbal epithelial stem cells are being activated and allowed to proliferate and migrate to close the epithelial defects and stimulating neuronal cells to proliferate and grow thus allowing normal neural innervations into the ocular tissue. It has been shown that IGF is lowered in the tears of diabetic patients. Furthermore, it is believed that IGF levels, epidermal growth factor and neuronal growth factors are elevated in the ocular tissue and tear fluid of these animals as a result of HGH application in these studies.

Recombinant human growth hormone (rHGH) is one example of an active agent related to HGH that can be effective as a treatment for a variety of ocular indications. Such indications can include, without limitation, the improved healing of ocular nerve and surface damages resulting from trauma, surgery, developmental defects, systemic and local disease, inflammatory process, and the like. rHGH can also be used in a preventative manner in cases where a subject may be developing or at risk for developing an ocular indication or condition.

As such, the present scope includes any active agent capable of increasing IGF or altering IGFBP that is capable of being formulated and delivered to the eye. In one aspect, for example, the active agent is HGH. In another aspect, the active agent is rHGH. In yet another aspect, the active agent is an HGH mimic (or rHGH mimic).

HGH mimics can include any active agent capable of producing substantially similar effects on IGF and/or IGFBP as compared to HGH. In one aspect, for example, an HGH mimic can be an animal HGH. In another aspect, an HGH mimic can be a mammalian non-human HGH mimic (e.g., bovine or porcine growth hormone). In yet another aspect, an HGH mimic can be a recombinant HGH (e.g. 191 amino acids). In other aspects, an HGH mimic can be an HGH structural variant (e.g., placental human growth hormone, a peptide derived from HGH, or the like). HGH can also be described as somatotropin or genotropin. Furthermore, rHGH can be obtained commercially from a number of sources including, but not limited to, Ferring Pharmaceuticals (Saint-Prex, Switzerland), Eli Lilly (Indianapolis, ID), and Pfizer (Groton, CT).

One example of HGH (e.g., human recombinant HGH) can have the following amino acid sequence (SEQ ID NO:1): see also e.g., US patents 6,136,563; 5,849,535; and 5,534,617, which disclose human growth hormone variants. Other HGH mimics can include structural variants such as placental human growth hormone Lacroix MC et al. Placenta. 2002 Apr;23 Suppl A:S87-94. One example of a human placental growth hormone has the following amino acid sequence (SEQ ID NO:2):

MAAGSRTSLL LAFGLLCLSW LQEGSAFPTI PLSRLFDNAM LRARRLYQLA
YDTYQEFEEA YILKEQKYSF LQNPQTSLCF SESIPTPSNR VKTQQKSNLE
LLRISLLLIQ SWLEPVQLLR SVFANSLVYG ASDSNVYRHL KDLEEGIQTL
MWRLEDGSPR TGQIFNQSYS KFDTKSHNDD ALLKNYGLLY CFRKDMDKVE
TFLRIVQCRS VEGSCGF

One example of porcine growth hormone has the following amino acid sequence (SEQ ID NO:3): 
FPAMPLSSLFANAVLRAQHLHQLAADTYKEFERAYIPEGQRYSIQNAQAAFCFSETIP
APTGKDEAQQRSDVELLRFSLLLIQSWLGPVQFLSRVFTNSLVFGTSDRVYEKLKDLE
EGIQALMRELEDGSPRAGQILKQTYDKFDTNLRSDDALLKNYGLLSCFKKDLHKAET
YLRVMKCRRFVESSCAF
 see e.g.,Vize & Wells Gene. 1987;55(2-3):339-44; US patent 5089473 which disclose mammalian (e.g., porcine) growth hormone variants.

One example of bovine growth hormone has the following amino acid sequence (SEQ ID NO:4): 
AFPAMSLSGLFANAVLRAQHLHQLAADTFKEFERTYIPEGQRYSIQNTQVAFCFSETI
PAPTGKNEAQQKSDLELLRISLLLIQSWLGPLQFLSRVFTNSLVFGTSDRVYEKLKDL
EEGILALMRELEDGTPRAGQILKQTYDKFDTNMRSDDALLKNYGLLSCFRKDLHKTE
TYLRVMKCRRFGEASCAF
 see also e.g., US patent 7101981 and WO/1988/007084 (which disclose bovine growth hormone variants). In a further aspect, an HGH mimic can be a human growth hormone secretagogue. HGH secretagogues are molecules that stimulate secretion or production of human growth hormone. Examples of HGH secretagogues include, but are not limited to, 4-(hydroxybutyl)carbamic acid 2-{5-[1-(2-amino-2-methylpropionylamino)-2-benzyloxyethyl]tetrazol-1-yl}ethyl ester, TZP-101, (+)-6-carbamoyl-3-(2-chlorophenyl)-(2-diethylaminoethyl)-4-trifluoromethyloxindole, and (±)-6-carbamoyl-3-(2, 4-dichlorophenyl)-(2-diethylaminoethyl)-4-trifluoromethyloxindole), ghrelin, or a ghrelin receptor agonist.

In one aspect, HGH and/or HGH mimics can be produced recombinantly.

Thus, the system described herein is useful in particular for treating or preventing neurotrophic dermal injuries and corneal injuries especially associated with trauma, diabetes, surgery, infections, and both systemic and ocular disease. Improvements of the levels and activity of IGF in the eye and dermal surface, through the mechanism of application HGH, remarkably improves epidermal and epithelial injury and improves neuronal innervations and sensation that decrease tissue breakdown and recurrence of disease. Furthermore, the results described herein indicate that delayed wound healing can be prevented through application of HGH. In particular, it was found that treatment of animals with a steroid *e.g.,* dexamethasone, delayed wound healing and this delayed healing was restored and revered through treatment with HGH.

An active agent such as HGH can be delivered to an eye of a subject via a variety of mechanisms and delivery modalities, both invasive and non-invasive. For example, in one aspect the active agent can be delivered to the eye in a topical form. Topical delivery can be in a frequently applied manner and/or in a sustained release manner that can facilitate improvements in healing and nerve re-innervation of ocular conditions such as, for example, diabetic neuropathic corneas and chronic herpetic keratitis, through restoration of improved and/or healthy corneal innervations. In such cases, a normal balance of tear film secretions and/or the heath of the ocular surface can be restored. HGH can also be used to treat recurrent corneal epithelial erosions, severe dry eye with epithelial defects, post-surgical corneal defects (i.e. refractive surgery or crosslinking surgery for keratoconus), chemical corneal burns, aseptic corneal perforations, and traumatic corneal and conjunctival injuries where the underlying pathology if impaired and traumatized corneal innervations (neurotrophic corneal pathology).

The use of HGH in a sustained release delivery mode can also allow the resurfacing of an artificial cornea and a corneal transplant by encouraging the proliferation and migration of endogenous already present corneal limbal epithelial cells and regeneration of corneal stromal innervation. Daily topical and /or sustained application of HGH can also up regulate and cross talk with sufficient growth factors to allow corneal epithelial cell proliferation and corneal neural cell migration from grafted limbal stem cells, with epithelial cells including cells that are derived from pluripotent stem cell grafts and amniotic tissue.

As has been described, delivery of an active agent to the eye can be accomplished via non-invasive or invasive techniques. An invasive technique is defined herein as an ocular delivery technique whereby an ocular membrane or tissue is physically disrupted during active agent delivery, or placement of an active agent depot. For example, injections, implantations, and the like are considered to be invasive because ocular tissue is disrupted by a needle or other surgical instrument during delivery. In topical delivery, on the other hand, an active agent is placed on a tissue surface of the eye and passively delivered therethrough. It should be noted that, the type of disruption caused by an electrical field such as by electroporation or iontophoresis would be considered to be a non-invasive delivery as such techniques generally do not physically disrupt ocular membranes and tissue. Microneedle delivery, on the other hand, would be considered to be an invasive technique.

The present technology is thus directed to systems and methods for sustained delivery of an active agent selected from HGH, rHGH, an rHGH mimic, or a combination thereof to the eye of a subject. It is noted that, for convenience sake, rHGH will be described throughout much of the remaining disclosure. The active agent is HGH, rHGH, or an HGH mimic. In a particular aspect, the polymer matrix with the formulation dispersed therein provides controlled release of an amount of the HGH to the eye effective to promote healing of a corneal wound due to underlying corneal de-innervation. As used herein in relation to the ocular nerve or corneal neural, "wound" refers to a defect in the cellular neural structure of the ocular tissue, regardless of whether the defect occurred from injury, disease, development, or human action.

The delivery approach according to aspects of the present disclosure enables a system including a drug-and-polymer depot to be placed in contact with tissues of the eye such that the active agent is released to the surface of the eye in a continuous or pulsatile manner, or is released into the eye at an internal location in a continuous or pulsatile manner. As used herein, the term "depot" refers to a collection of material that includes an active agent and that can be placed in an area of interest to provide sustained release of the active agent at least to that area. Accordingly, a method of promoting healing of ocular nerve damage in a subject can include placing an active agent delivery depot as described herein in an eye of the subject. The composition can be placed in a variety of locations on or in the eye, and any such location is considered to be within the present scope. In one aspect, for example, a depot can be placed adjacent to a surface of the cornea, conjunctiva, and or sclera. Placement of the depot can also be on or within the sclera (episcleral); beneath or within overlying tissues such as the subconjunctival tissue, e.g. at or near the limbus; the periocular region; within the sub-Tenon's space; and in posterior retrobulbar locations.

The processes of cell growth and proliferation involved in healing of ocular nerve damages can be ongoing for some time before healing is complete. During that time, the rate and efficiency of these processes can depend on the ability to maintain at least a minimum titer of HGH or other active agent over the healing period. Drug delivery duration will depend upon the severity and underlying process being treated. In one aspect, the composition and location of the composition can be selected to allow the controlled and sustained release of HGH to occur over a span of from several hours to several months. In a specific example, the depot provides controlled release for a period from about 2 days to about 200 days. In another aspect, the controlled release has a duration of from about 1 hour to about 200 days. In yet another aspect, the drug-polymer depot can be configured to provide continuous release having zero-order kinetics over substantially the entire release duration.

Release by the composition provides a dose of HGH to the eye in which it is placed. In one embodiment, the HGH is released in a continuous fashion for a particular duration. In alternative embodiment, the composition provides release of HGH in a pulsatile fashion, i.e. two or more discrete doses of a given duration and amount and separated by an interval of time. The timing of the pulses can be according to a single fundamental frequency, or can exhibit a more complex temporal pattern. This allows for an additional level of control of release, e.g. to promote greater efficacy or address safety issues. For example, intermittent release can reduce potential adverse effects of constant HGH stimulation, which may prevent such inactivation or down-regulation of receptors. In another aspect, a pulsatile delivery can be used to simulate and allow a natural course of release of endogenous growth hormone.

Controlled release by the composition provides to the eye a dose of HGH that is effective to promote healing of ocular nerve de innervations, damage and or defects. In one embodiment, the composition is configured to release a particular amount of HGH per day. An effective amount of HGH may depend on the type of wound or its etiology. Other possible factors include the age, weight, and medical history of the subject. Accordingly, the composition can be configured to provide a proper dose based on these or other factors. In one example, the composition can provide from about 0.04 mg to about 4.0 mg of HGH per kg of the subject's body weight. In another example, release of HGH can be upwards of 250 mg for a 60 day delivery. In another example, the concentration of HGH included in the composition polymer material is from about 0.1 µg/ml to about 100 µg/ml. In one aspect, the amount of HGH provides a concentration of about 0.01% to about 0.5% HGH in a 30-50 µl eye drop administered QID (4x/day). In still another aspect, 100 ug/ ml can be topically delivered in a 30 - 50 µl dose 4 x/ day to heal rabbit corneas. For example, a 12 µg loading dose can release 4-6 µg upon placement with 1-2 µg/ day thereafter for up to 1 week. In another aspect, the total daily concentration of HGH provided is from about 0.2% to about 2.0% or 0.5 µg to 10 µg/day.

HGH is combined with a polymer matrix, and an amount of this combination is used to create a drug-polymer composition that provides controlled release of HGH. The physical properties of the composition can be selected to be suitable for different modes of placement, e.g. topical application on the surface of the eye or subconjunctival, subtenons, peribulbar placement. The drug-polymer composition can comprise a microparticle or nanoparticle suspension, a solid or semi-rigid monolithic rod, film or a gel. In one embodiment, the polymer matrix can be sufficiently liquid to be administered as eye drops and then allowed to gel on the surface. In another aspect, the polymer matrix can be injected into an ocular space such as the subconjunctival space as a liquid and or gel. In still another aspect, the drug-polymer matrix can be applied to a structure that is then placed on an ocular surface in the cul de sac and or on the cornea. With such approaches, the polymer matrix can be selected to be flowable while exhibiting sufficient cohesiveness so that it is not easily diluted or washed away from the placement site. In another embodiment, the polymer matrix can be selected to form a more solid structure shaped for placement on or under an ocular surface.

In a particular embodiment, the composition can comprise polymers that are bioerodible, so that the composition is gradually broken down over time rather than needing to be removed at the end of a treatment period. As used herein, "bioerodible" refers to the ability of a material to be broken down by processes in a physiological environment, and rendered into smaller units that can be dealt with by the body. In particular this can refer to rendering the material water-soluble and further resorbable by the body. In one embodiment, controlled release of the active agents from the composition is accomplished by the degradation of bioerodible biopolymers included in the polymer matrix.

The polymer matrix is a bioerodible polymer comprising a cross-linked hyaluronic acid of a thiolated carboxymethyl hyaluronic acid cross-linked with poly(ethylene glycol) diacrylate. In accordance with the present disclosure, a drug delivery system can utilize other mechanisms for controlled release of active agent formulation. For example, the drug-polymer matrix can be substantially contained in a space under a structure on the ocular surface, such as a contact lens or bandage lens. The composition may be applied to the underside of the contact lens before insertion in the eye, or alternatively the composition can be applied to the cornea or sclera and subsequently covered by the lens.

In another embodiment, the system can include a structure to mediate release of the formulation to the eye. In a particular example, the composition can be placed adjacent to a rate controlling diffusion barrier that comprises diffusion control materials, e.g. in a subconjunctival implant. In another example of an implant, release can be aided or accomplished by iontophoresis. The implant can include a membrane or barrier having transport properties that are modulated by changing the electrical state of the barrier. Non-limiting examples of electrically inducible mechanisms for drug release include ion exchange and electroporation. Iontophoretic release can be controlled by application of a signal to the drug delivery system. Such a control signal, e.g. an electrical signal, can be applied directly to the implant, or alternatively can be conveyed by a remote signaling device. To accommodate this type of control, the implant can further include a device, e.g. a microchip, configured to receive and transmit a signal to the barrier that is appropriate to modify the electrical state of the barrier. Additionally, it is also contemplated that an implant can utilize an expanding hydrogel to deliver the active agent from the implant reservoir.

In addition to HGH, the formulation dispersed in the polymer matrix can include other suitable active agents. These secondary active agents can promote neural ocular and corneal regeneration, either independently or in conjunction with the primary active agent (e.g. rHGH). Alternatively, secondary active agents having other effects on the condition of the eye can be included. In keeping with the indication of HGH for wound healing, additional active agents can be chosen that will not interfere with this action of HGH. Suitable active agents for inclusion can include by way of example:
Antibiotics such as ciprofloxacin, gatifloxicin, moxifloxacin, bacitracin, tobramycin, macrolides, polymyxin, gramidicin, erythromycin, tetracycline, azithromycin, erythromycin, and the like;
Anti-inflammatory agents such as hydrocortisone, dexamethasone, triamcinolone, prednisolone, fluorometholone, flucinolone acetate, loteprednol etabonate, and medrysone;
Non-steroidal anti-inflammatory drugs such as flurbiprofen sodium, diclofenac sodium, ketorolac, indomethacin, and ketoprofen;
Analgesics such as lidocaine and tetracaine;
Antivirals such as ganciclovir;
Antifibrotic such as but not limited to anti TGF beta drugs, IL modulators, and TK inhibitors;
Glaucoma agents such as, but not limited to, Lanatoprost, Bimatopost, Travaprost, Timolol, Betaxalol, Dorzolamide, Brinzolamide, and Briminodine; and
Growth factors such as, but not limited to, basic fibroblast growth factor, epidermal growth factor, insulin like growth factor, hepatocyte growth factor, neuronal growth factor, and brain derived growth factor.

Other specific examples of secondary active agents can include, without limitation, Prokera, Prose, Ambiodisk, Ambiodry, amniotic membranes, Autologous serum, umbilical cord serum, and the like.

Secondary active agents can also include various fluoroquinolones, such as, but not limited to, besifloxacin, ciprofloxacin, levofloxacin, ofloxacin, moxifloxacin, gatifloxacin, and the like.

Other examples can include aminoglycosides such as tobramycin, gentamicin, and the like. Polymyxin B combinations such as polymyxin B/trimethoprim, polymyxin B/bacitracin, polymyxin B/neomycin/gramicidin, and the like.

It is noted that, while examples listed above are described using either their branded or generic names, the present scope is contemplated to include both branded and generic active agents and active agent equivalents.

Other additional active agents can include artificial tears solutions, cellular adhesion promoters, decongestants, anticholinesterases, glaucoma agents, anti -oxidants, cataract inhibiting drugs, antiallergenics, antioxidants, anti angiogenic drugs, as well as other drugs that may be indicated for use in the eye while not interfering with the action of HGH.

The formulation, including any of the secondary active agents or other components thereof, can be dispersed in a polymer matrix in any form that provides suitable stability and release kinetics. The forms in which the formulation is dispersed in the polymer include, without limitation, as a solid (rod, fiber and or thin film or strip) a gel, a powder, a suspension, and an emulsion. Thus, the composition can be shaped as an independent structure or can be associated with another substrate such as a coating on a contact lens and or punctual plug. Suspensions can be micro and/or nanoparticle suspensions. Similarly, the composition can be configured as a solid or semi-rigid monolith rod, a gel deposition with controlled degradation and release of the active drug. For delivery as a sub-conjunctival depot, iontophoretic structures can be included for assisting active migration of the drug into ocular tissue.

In a particular embodiment, the composition can be configured for a single use, where the polymer matrix and formulation are combined before placement and the composition or implant is removed or degrades upon exhaustion of the formulation. In an alternative embodiment, a formulation can be added to the polymer matrix after implantation, e.g. by injection. Injection can be made through overlying ocular structures (e.g. the conjunctiva in a subconjunctival implantation), or an injection port can be included that provides access to the polymer matrix.

In another aspect of the present disclosure, composition according to the invention is for use in preventing or improving delayed ocular wound healing in a subject. Delayed wound healing refers to wounds that do not heal at the expected rate. Delayed wound healing can result from a number of problems. One specific case of delayed wound healing occurs when treatment of a wounded tissue (from surgery or other insults such as, for example, physical trauma from chemical burns, infection, or the like) with a compound (*e.g*., steroid or corticosteroid (dexamethasone) and or immunosuppressant slows the healing of the wound compared to a similarly wounded tissue that was not treated with the compound. For example, diabetic corneal abrasions or surgical corneal defects that require steroids for inflammation control but have wound healing delay. Examples of steroids used for treating inflammation in ocular diseases or conditions, include, but are not limited to, topical dexamethasone (e.g., Dexasol®), Lotemax® (loteprednol ophthalmic suspension 0.5%, Bausch and Lomb), Durezol® (difluprednate ophthalmic emulsion 0.05%, Alcon), triamcinolone, medrysone, fluorometholone, and/or topical or oral prednisolone.

As used herein, an effective amount as it relates to wound healing is an amount of a therapeutic agent (e.g., rHGH) that when applied to a wounded tissue accelerates the rate of wound healing of a wounded tissue as compared to a similarly wounded tissue that is not treated with the therapeutic agent.

Other options for use of the compositions can include under and/or in conjunction with an amniotic membrane graph, corneal transplant, limbal stem cell transplant and or scleral flap during and used at the time of surgery. Further, using the compositions with a corneal transplant procedure, or any ocular surgery, optionally in conjunction with explants can be suitable. In addition, the compositions can be implanted with limbal stem cells and amniotic graph transplants. In yet another alternative, the device and compositions can be used immediately after a burn and/or traumatic chemical injury (i.e. mustard gas and alkali burns) to initiated the healing process and reverse and or lessen the amount of ischemic and chemical damage that is incurred if the insult goes untreated during transport.

In another aspect, the present disclosure additionally provides a variety of ocular inserts for the delivery of the active agent into the eye. An ocular insert is a sterile, thin, multilayered device and/or implant having a solid or semisolid consistency configured to be placed into the cul-de-sac or conjuctival sac and/or on the surface of the bulbar conjunctiva and or cornea, whose size and shape are designed for ophthalmic application. An ocular insert is composed of a polymeric support that may or may not contain an active agent. An ocular insert can be a soluble, bioerodible, or insoluble (e.g., osmotic, diffusion, or contact lens like). The active agent can be incorporated as a dispersion or a solution in the polymeric support or any other acceptable manner (e.g., as a coating on a contact lens). An ocular insert may be configured to have a body portion sized to position within the conjunctiva cul de sac of the eyelid. A few non-limiting examples of ocular inserts can include membrane-bound ocular inserts (biodegradable and non-biodegradable), for example, Ocuserts® (Alza Corp), mucoadhesives dosage forms (ocular films or sheath, ophthaCoil, polymer rods, HEMA hydrogel, dispersion, polysulfone capillary fiber), collagen shields, cyclodextrine-based systems, ophthalmic rods (artificial tear inserts, e.g., Lacrisert®), and the like.

In one aspect, the ocular insert as described herein can include a variety of useful properties. For example, an ocular insert can be made of a bioerodible film, and thus degrades in the subject's body (e.g., eye or cul-de-sac of the eye) from about 1 to about 30 days, from about 3 to about 20 days, from about 5 to about 14 days, and in some cases from about 7 to about about 10 days. Such an insert device can also release an active agent over the course of the degradation period, and is formable into various shapes, depending on the desired application. Additionally, inserts can be nonirritating to the patient, and drug released from the ocular insert remains active. Importantly, the ocular insert can be produced as a sterile final product.

The ocular insert, in some aspect, has a thickness which allows for use in the eye. For example, the hydrogel thickness can be from about 0.1 mm to about 5 mm thick, about 0.2 mm to about 5 mm thick, 0.3 about mm to about 5 mm thick, 0.4 about to about 5 mm thick, about 0.5 to about 4.5 mm thick, about 0.5 to about 4 mm thick, about 0.5 mm to about 3.5 mm thick, or about 0.5 to about 3 mm thick. As the skilled artisan understands, the specific dimensions of the ocular insert may vary although the size is commensurate with the specific application. A variety of shapes are contemplated herein for the ocular insert including, but not limited to, discs and threads. The insert can be of any shape and size and, preferably, is in the shape of a rod, strip, thread, doughnut, disc, oval, or quarter moon. It can be so large as to cover the entire globe of the eye or small enough to be inserted between the glob and the superior and/or inferior lid as well as against the cornea and or sub conjunctively and or subtenons. In one aspect, the ocular insert is provided as a sterile, single-dose (e.g., controlled released), ophthalmic formulation.

The ocular insert is a hydrogel comprising a thiolated carboxymethyl hyaluronic acid cross-linked with a poly(ethylene glycol) diacrylate. In one aspect, the hydrogel is a hyaluronic acid moiety cross-linked with a polyalkylene diacrylate moiety. In one aspect, the hydrogel is a hyaluronic acid moiety cross-linked with a polyalkylene diacrylate moiety wherein the polyalkylene portion of the diacrylate is an alkylene group having from 1 to 4 carbons.

In one embodiment, the ocular insert is a hydrogel comprising a thiolated carboxymethyl hyaluronic acid cross-linked with poly(ethylene glycol) diacrylate wherein the ratio of thiol to acryl is from 1:2 to 6:1, 1:1 to 5:1, 1:1 to 4:1, or 1:1 to 3:1. In one embodiment, the ocular insert is prepared from a thiolated carboxymethyl hyaluronic acid (CMHA-S) using from about 5 to about 25, about 7 to about 22, about 10 to about 19, or about 11 to about 17 mg/ml and a second moiety which is a polyalkylene diacrylate. In one aspect of this embodiment, wherein the polyalkylene diacrylate is poly(ethylene glycol) diacrylate. In another aspect, the ocular insert is a films containing thiolated carboxymethyl hyaluronic acid cross-linked with a poly(ethylene glycol) diacrylate to have from 4:1 to 1:2 or about a 1.5:1 thiol:acryl (or plus or minus 20% thiol or diacrylate), and also contains from about 1 mg/mL to about 50 mg/mL or 10 mg/ml (or plus or minus 50 %) methylcellulose (MC). The MC provides some flexibility to the films and renders them somewhat mucoadhesive. Hyaluronic acid and derivatives thereof can form hydrogels with a variety of molecules including, but not limited to, dithiobis(propanoic dihydrazide) (DTP), dithiobis(butyric dihydrazide), and the like.

Hyaluronic acid and derivatives thereof can form hydrogels with a variety of other molecules by crosslinking with a poly(ethylene glycol) diepoxide group. The hydrogel may further include other agents. One example of such agents is a mucoadhesive and/or flexibility improving agent *e.g*., methylcellulose. The other agents in the hydrogel are chosen such they do not prevent formation of the hydrogel or release of the drug from the hydrogel. Agents that can improve flexibility and/or mucoadhesiveness of the ocular insert are known to the skilled artisan.

As described herein, the present technology provides an ocular insert comprising HGH. As has been noted above, the term "HGH" mimic includes any compound that mimics HGH activity in treating or preventing PCED or delayed wound healing, and such compounds are contemplated to be used. In one specific aspect, the HGH mimic activates or increases IGF (and/or alters IGFBP) in ocular tissue or tears. HGH mimics can be human growth hormone homologs from other animals like mammals (*e.g.*, the bovine or porcine homolog of human growth hormone). The HGH mimic can be human HGH that is modified. The HGH mimic can be a human rHGH homolog having from 1 to 30 amino acid substitutions wherein the amino acid substitutions are conservative substitutions. The HGH mimic can be a fragment of HGH having at least about 10, about 15, about 20, or about 30 or more contiguous amino acids of HGH (e.g., HGH variants). The HGH mimic can be an HGH peptidomimetic. The HGH mimic can be a preparation of HGH derived from human tissue or cells. The HGH mimic may be an agent that increases HGH in ocular tissue or ocular tears. In some embodiments, the ocular insert can have HGH incorporated into the hydrogel at from about 0.05 to about 100, about 0.1 to about 50, about 0.2 to about 25, or about 1 to about 20 mg/mL.

The topical ocular pharmaceutical compositions can include a topical ocular carrier and an effective amount of an active agent (*e.g.,* rHGH or an rHGH mimic). The pharmaceutical compositions described herein may comprise a carrier suitable for intraocular administration, such as, for example, Ringer's solution or balanced salt solution, and an effective amount of a pharmaceutically acceptable HGH. The carrier for intraocular pharmaceutical compositions is preferably free of microbials and endotoxins. Topical ocular formulations provided herein are configured for application to the eye. In some embodiments, the topical ocular formulation is in the form of ocular inserts, eye drops, eye washes, contact lens solutions, ointments, gels, patches, packs, depot formulations, sustained or continued release formulations, aerosols, and the like. In various embodiments, the topical ocular formulation is provided in single or multi-dose containers or dispensers. The disclosure also provides intraocular formulations in the form of injectable solutions, eye irrigating solutions, volume replacement solutions, films, gels, depot formulations, slow release formulations, and the like. In various embodiments, the intraocular formulation may be provided in single or multi-dose containers or dispensers, or in implantable intraocular devices.

An article of manufacture is also provided herein. According to one embodiment, the article of manufacturer comprises a vessel containing a composition or formulation as described herein and instructions for use for the treatment of a subject. For example, an article of manufacture, comprising a vessel containing a formulation configured for topical application to the eye and instructions for use for the treatment of a subject suffering from a chronic, delayed healing, or incomplete healing wound, or other wound that does not heal at an expected rate is provided and or a wound that would not be expected to heal at the appropriate rate even prior to creation of the defect.

In the present description, the terms "topical" and "topical application" refer to the non-systemical administration of the active ingredient to the external surface of the wound for local effect. Preferably the composition is sterile or aseptic and can be packaged in tubes, bottles or other containers suitable for easy topical application. The pharmaceutical composition of the present invention comprises a formulation including an active agent that increases insulin growth factor or that alters insulin growth factor binding protein in a subject is dispersed in a pharmaceutical carrier, wherein the active agent is HGH, rHGH, an rHGH mimic, or a combination thereof, wherein the composition is configured for placement in or on an eye of the subject, wherein the composition provides controlled release of an amount of the active agent to the eye effective to promote at least one of ocular surface regeneration, corneal epithelial regeneration, and neural regeneration, and the pharmaceutical carrier is a polymer matrix of a bioerodible polymer comprising a cross- linked hyaluronic acid of a thiolated carboxymethyl hyaluronic acid cross-linked with poly(ethylene glycol) diacrylate. In one aspespect of this embodiment, the active agent is rHGH. In one aspect of this embodiment, the drug delivery composition is configured for administration to a subconjunctival location. In another aspect of this embodiment, the drug delivery composition is configured for injection to the subconjunctival location. In yet another aspect of this embodiment, the drug delivery composition is configured for administration to an ocular location which is the cul de sac, conjunctiva, cornea, subconjunctival limbus, subscleral, intra corneal, periocular region, subtenon's space, or retrobulbar space. The composition can have a controlled release with a duration of from about 1 hours to about 200 days. In one aspect of this embodiment, the controlled release exhibits zero-order and/or near zero order, with and/or without a burst, kinetics for substantially the entire duration. In one aspect of this embodiment, the active agent is released as a number of pulsed doses. In one alternative aspect, the active agent is released continuously. The drug delivery composition can be configured for administration under a contact lens or bandage lens worn in the eye. As on example, the composition can provide continuous release of rHGH for at least 3 days, at least 4 days, at least 5 days, at least 6 day or at least 7 days.

The composition can also be formulated as a microparticle suspension, a nanoparticle suspension, a monolithic rod, film, a gel, or a combination thereof. Generally, the composition can be formulated as an ocular insert, an implantable depot, a daily topical formulation, a spray formulation, a sustained topical formulation, an injectable fluid, a gel, a film, a sponge, or an ointment.

Additional components can be optionally included. For example, the bioerodible polymer can further include an ingredient that increases mucoadhesiveness or flexibility. In one aspect of this embodiment, the ingredient that increases mucoadhesiveness of flexibility is methylcellulose. In one aspect of this embodiment, the formulation further includes a second bioactive agent selected from antibiotics, antimicrobials (antivirals or antifungals), anti-inflammatory steroids, non-steroidal anti-inflammatory drugs, analgesics, artificial tears solutions, cellular adhesion promoters, growth factors, decongestants, anticholinesterases, antiglaucoma agents, cataract inhibiting drugs, antioxidants, anti-angiogenic drugs, antiallergenics, or a combination thereof. In one aspect of this embodiment, the second bioactive agent is an antibiotic selected from ciprofloxacin, gatifloxicin, moxifloxacin, bacitracin, tobramycin, macrolides, polymyxin, gramidicin, erythromycin, tetracycline, or a combination thereof. In one aspect of this embodiment, the second bioactive agent is an anti-inflammatory steroid selected from the group consisting of hydrocortisone, dexamethasone, triamcinolone, prednisolone, fluorometholone, flucinolone acetate, medrysone, or a combination thereof. In one aspect of this embodiment, the second bioactive agent is a non-steroidal anti-inflammatory drug selected from flurbiprofen sodium, diclofenac sodium, ketorolac, indomethacin, ketoprofen, or a combination thereof. In one aspect of this embodiment, the second bioactive agent is an analgesic selected from lidocaine, tetracaine, or a combination thereof.

In one embodiment, a drug delivery composition comprising a formulation including an active agent that is HGH, rHGH, an rHGH mimic, or a combination thereof, dispersed in a polymer matrix for preventing or improving delayed ocular wound healing in a subject is provided. The polymer matrix can provide controlled release of an amount of the active agent to the eye effective to prevent or improve delayed wound healing. In one aspect of this embodiment, the active agent is rHGH. In one aspect of this embodiment, the composition is for preventing or improving delayed ocular wound healing wherein the subject is expected to have a delayed wound healing and or decreased innervation condition of the eye. In one aspect of this embodiment, the delayed wound healing condition is induced, caused by, or associated with treatment of the subject with another drug. In one aspect of this embodiment, the another drug is a steroid. In one aspect of this embodiment, the another drug includes a member selected from hydrocortisone, dexamethasone, triamcinolone, prednisolone, fluorometholone, flucinolone acetate, medrysone, or a combination thereof. In one aspect of this embodiment, the drug delivery system is an ocular insert, solution, film, or gel. In one aspect of this embodiment, the drug delivery system is an ocular insert or gel comprised of a bioerodible polymer comprising a moiety derived from thiolated carboxymethyl hyaluronic acid and a moiety derived from poly(ethylene glycol) diacrylate. In another aspect, the concentration of the active agent (e.g., rHGH) in the matrix is from about 0.05 µg to about 100 µg per milliliter. In one aspect of this embodiment, the composition provides continuous release of rHGH for at least 3 days, at least 4 day, at least 5 days, at least 6 day or at least 7 days. The drug delivery composition can be configured for administration to a subconjunctival location. In one aspect of this embodiment, the drug delivery composition is configured for injection and/or placement into the subconjunctival and or subtenons location. In one aspect of this embodiment, the drug delivery composition can be configured for administration to an ocular location selected from the cul de sac, conjunctiva, cornea, subconjunctival limbus, subscleral, intra corneal, periocular region, sub-Tenon's space, or retrobulbar space. In one aspect of this embodiment, the controlled release has a duration from about 1 hours to about 200 days. In one aspect of this embodiment, the controlled release exhibits zero-order and or near zero order with and or without a burst, kinetics for substantially the entire duration. In one aspect of this embodiment, the active agent is released as a number of pulsed doses. In one aspect of this embodiment, the active agent is released continuously. In one aspect of this embodiment, the drug delivery composition under is configured for administration under a contact lens or bandage lens worn in the eye.

In one embodiment, a composition for continuous delivery of rHGH to the eye comprises rHGH in a polymeric matrix of a bioerodible polymer comprising a cross- linked hyaluronic acid of a thiolated carboxymethyl hyaluronic acid cross-linked with poly(ethylene glycol) diacrylate, for use in preventing or improving delayed ocular wound healing in a subject is provided. In one aspect of this embodiment, composition is an ocular insert, solution, film or gel. In one aspect of this embodiment, the composition is for preventing or improving delayed ocular wound healing in a subject wherein the delayed ocular wound healing in the subject is caused by or associated with treatment of the subject with another drug. In one aspect of this embodiment, another drug is a steroid. In another aspect, the concentration of the active agent (*e.g.,* rHGH) in the matrix is from about 0.05 µg to about 100 µg per milliliter. In one aspect of this embodiment, the composition is an ocular insert from about 0.1 mm to about 5 mm thick, about 0.2 mm to about 5 mm thick, 0.3 about mm to about 5 mm thick, 0.4 about to about 5 mm thick, about 0.5 to about 4.5 mm thick, about 0.5 to about 4 mm thick, about 0.5 mm to about 3.5 mm thick, or about 0.5 to about 3 mm thick. In one aspect of this embodiment, the composition provides continuous release of rHGH for at least 3 days, at least 4 day, at least 5 days, at least 6 day or at least 7 days.

### Examples

### Example 1: Wound Healing of the Eye

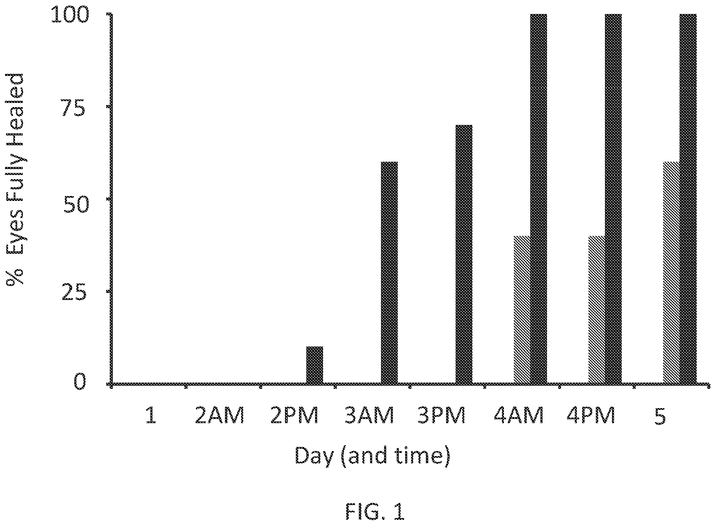

A rabbit debridement model was used to assess the effect of topically applied rHGH on corneal epithelial defect closure in a model where epithelial closure was impaired by concomitantly administered topical steroids (Dexamethasone 0.1% QID). Standard epithelial defects were created in the eyes of nine New Zealand white rabbits by standard methods. Rabbits received topical dexamethasone QID (4 x/day) to all 18 eyes. Rabbits were randomized to one of three treatment arms post surgery: a control arm of dexamethasone only QID OU (both eyes), a vehicle group of BSS (balanced salt solution) QID OU, and test article of rHGH QID OU. One drop (50 µL) of rHGH (reconstituted in Ringers Lactate to achieve a final concentration of 100 µg/mL) (n=3) was applied topically four times to both eyes. All eyes were also treated with one drop of an antibiotic solution after the surgical defect creation. Photographic assessment (twice daily) and fluorescein assessment were used to determine the speed and quality of epithelial defect closure. The corneal defect size was estimated in the photographs by obtaining the pixel count of the defect and dividing by the pixel count of the cornea. The animals were sacrificed on day 5. Comparison of epithelial defect size between control (BSS) and the rHGH-treated eyes showed more rapid epithelial closure when assessed photographically in the epithelial defects treated with rHGH 100 µg/mL. As shown in FIG. 1, the dexamethasone-treated eyes alone healed the slowest with no epithelial defects completely closed by day 5 (0/9) (FIG. 1 - no bar since no healing was achieved), followed by the BSS (FIG. 1 - shaded bars) where only 50 % eyes were healed by day 5 (4.5/9) and lastly with the rHGH topically administered (FIG. 1 - black bars) eyes showing the most rapid healing in that all the eyes with 9/9 eyes healed by day 5 and 10 % of the eyes starting to heal as quickly as Day 2. The animals tolerated the dosing well without clinical concerns.

Specifically, FIG. 1 shows the effect of rHGH on wound healing of the cornea with the y-axis representing the amount of eyes completely healed as a function of time (x-axis in days). The solid black bars represent percent of eyes completely healed when treated with rHGH administered topically whereas the shaded bars represent percent of eyes completely healed when treated with BSS (balanced saline solution). Over the course of this study, no eyes were completely healed when treated with dexamethasone alone.

### Example 2: Preparation of rHGH-Polymer Topical Formulation

1) HYSTEM-LS vial (20 mg) {thiolated hyaluronate (e.g., thiolated carboxymethyl hyaluronic acid)} (store at -20 deg C for long term storage; if resuspended, store at room temperature for 12 hours maximum). Quantity: 10. Resuspend one each day and use as stock for that day. Once resuspended, use throughout day. Do not refreeze remainder for next day.
2) Lactated Ringers vial (10 ml, store at room temp). Store at 4 deg C. Quantity: 5.
3) 20 mM oxidized glutathione (GSSG) in Lactated Ringers (0.0656 g in 5 ml Lactated Ringers) (Sigma-Aldrich catalog number: G4626) (store powder at -20 deg C; store resuspended solutions at -20 deg C). Quantity of vials: 2. These are resuspended and aliquoted out on surgery day.

### GSSG:

Step 1: To the tube filled with GSSG powder, swab top of lactated ringers vial with an alcohol wipe and withdraw 5 ml Lactated Ringers solution. Add 5.0 ml to GSSG tube and vortex well to resuspend. Be sure all powder is dissolved.
Step 2: Affix 0.2 micron syringe filter to a 5.0 ml syringe with its plunger pulled out. With the syringe filter still in its container, fill syringe with 5 ml GSSG solution. Using plunger, filter sterilize into a fresh sterile 15 ml falcon tube. Recap the tube.
Step 3: Aliquot into sterile eppendorf tubes:
   a) 50 ul/tube, 20 tubes (this is for the HYSTEM/GSSG administrations)
   b) 125 ul/tube, 20 tubes (this is for the HYSTEM/GSSG/hGH administrations)
   c) Label tubes as "50 ul" or "125 ul" and place all tubes at -20 deg. C for the week.

### HYSTEM-LS:

Step 4: To one HYSTEM-LS vial, take off blue cap and swab top of vial with alcohol wipe. Also swab the top of the Lactated Ringers vial. With a sterile syringe, withdraw 2 ml Lactated Ringers Solution and inject into the HYSTEM-LS vial.
Step 5: flick vigorously with finger to make sure pellets are completely hydrated.
Step 6: Mechanical agitation by hand or by rotary shaker (150 rpm with tube on side) until pellets have completely dissolved (up to 45 min or until all translucent chunks have dissolved).
Step 7: Carefully remove metal crimp on HYSTEM-LS vial with pliers.
Step 8: Leave at room temperature on the lab bench for the day (at end of day, discard any HYSTEM-LS not used).

### Genotropin (hGH):

Step 9: 20X stock (800 ug/ml) for HYSTEM-LS/GSSG mixture: Carefully remove lyophilized cake of Genotropin to a 15 ml conical tube using sterile forceps.
Step 10: Resuspend gently by swirling tube by hand in 2.5 ml Lactated Ringers and place at 4 deg. C for the day. (2.5 ml total volume).

HYSTEM-LS/GSSG/Genotropin Application: (Note: start preparing solutions 1 hr before animals are ready)

### Prior to Application:

Step 11: Thaw a "125 ul" GSSG tube in your hands.
Step 12: Add 469 ul of HYSTEM-LS to the GSSG tube. Mix by upending tube.
Step 13: Add 31 ul 20X Genotropin stock and mix gently by upending well by hand (no vortexing). (Note: Gelation occurs in about 10 minutes at room temperature).
Step 14: With a sterile P200 tip, aspirate 50 microliters of the mixture and gently dispense onto surface of the 10 rabbit eyes randomized to the test HYSTEM/rHGH group. Make sure it coats the rabbits eye completely (manually blink / close the eye lids if possible to distribute the solution over the ocular surface). This tube will supply all 10 eyes for the active test control group
Step 15: Discard HYSTEM/GSSG/Genotropin tube when done.
Step 16: 20X stock can be saved at 4 deg C for subsequent days.

Alternative topical formulations containing polymerized crosslinked hyaluronic acid (HYSTEM/ GSSG) and rHGH can be formulated and used in view of the above by one of ordinary skill in the art.

### Example 3: Wound Healing of the Eye

A corneal epithelial impaired healing debridement model was developed by utilizing a validated epithelial debridement procedure and treating immediately postoperative with topical steroids (dexamethasone). The corneal epithelial defects were created surgically in a standardized and reproducible manner in both eyes. The normal healing for epithelial defects in normal healthy rabbits is 4 - 5 days. With the addition of topical dexamethasone (0.1% administered in all eyes starting immediately post defect creation, 4 x/day for 7 days), the healing was delayed out to 7 days and there were still rabbits even at 7 days who did not have complete epithelial defect closure. This model confirmed delayed epithelial wound healing in New Zealand white rabbits.
Rabbits were randomized post surgical intervention to maintain a masked study. The examiner was masked to the treatment arm.

Recombinant Human Growth Hormone (rHGH) was reconstituted and prepared as instructed, and was delivered topically to the eye via a crosslinked hyaluronic acid gel polymer HYSTEM (with glutathione GSSG) at a concentration of 4 ug/day in a 50 ul drop BID, for 7 days (see Example 2). The comparator arms are shown in Table 1: (1) the polymer HYSTEM with GSSG but without rHGH (group 1) and (2) dexamethasone alone (no polymer and no rHGH) (group 3). *In vivo* clinical exams were performed twice daily to determine percent corneal healing, corneal defect size, and percent of eyes (cornea) completely healed.

**Table 1**

| Group | Treatment | Animals N= (Eyes) | Exam schedule | Exams |
|---|---|---|---|---|
| 1 | HYSTEM (with GSSG) alone + dexamethasone | 2 (4) | 10 exams total per rabbit per eye | Slit lamp biomicroscopy, flourescein staining, and digital photographs Assessment of corneal defects via Image J |
| 2 | HYSTEM (with GSSG) + rHGH + dexamethasone | 5 (10) | Day 1: 3 exams, including baseline after wound creation | |
| 3 | Control (dexamethasone only) | 4 (8) | Day 2, 3, & 4: 2 exams each day | |
| | | | Day 5, Day 6, and Day 7: 1 exam each day (AM) | |

HYSTEM/GSSG+rHGH (group 2) showed the most rapid corneal wound healing rate when compared to the other test groups as measured by percent corneal healing each day. Comparing the HYSTEM/GSSG (group 1) and HYSTEM/GSSG+rHGH (group 2) treatments versus the dexamethasone alone (group 3), the HYSTEM/GSSG+rHGH (group 2) showed increased corneal healing rates.

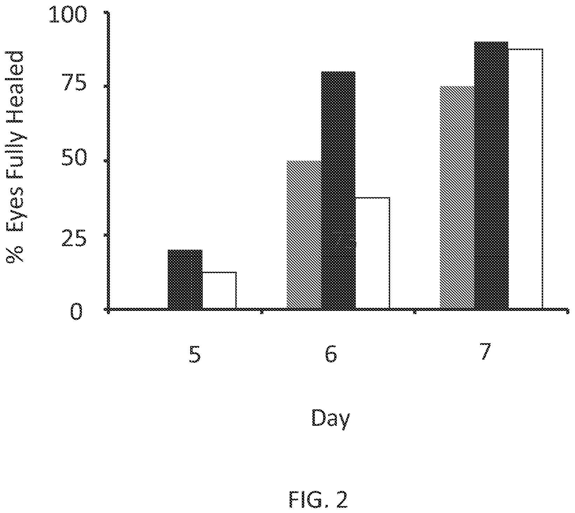

Percentage of eyes completely healed over the course of the study is shown in FIG. 2. The eyes treated with HYSTEM/GSSG+rHGH+dexamethasone (group 2 - black bars) showed the most rapid positive change in accelerated corneal healing with the greatest percent of eyes completely healed as early as day 5, (group 3 (dexamethasone) and group 1 shaded bars (grey) (HYSTEM/ GSSG + dexamethasone)) . Analysis of changes in corneal defect size (mm) indicated that the HYSTEM/GSSG+rHGH had the fastest rate of decreased corneal defect size and closure.

The HYSTEM/GSSG+rHGH+dexamethasone (group 2) again showed the fastest decrease in corneal defect size over the 7 day period. All test articles were very well tolerated during the in life portion of the study. In terms of efficacy, the HYSTEM/GSSG + rHGH + dexamethasone (group 2) showed a positive signal in accelerating corneal wound healing in this corneal debridement model. The use of the topical steroid, dexamethasone, applied to all arms was intended to slow down the corneal time to complete re-epithelization. The HYSTEM/GSSG + rHGH test article (group 2) appears to be the most efficacious of the formulations used this study. These studies showed that impairment of corneal wound healing (*e.g.,* by dexamethasone) was improved and restored by the rHGH administered through the HYSTEM/GSSG polymer.

Specifically, FIG. 2 shows the effect of rHGH in a polymer gel formulation on wound healing of the cornea with the y-axis representing the amount of eyes completely healed as a function of time (x-axis in days). The solid black bars represent percent of eyes completely healed when treated with the rHGH polymer gel formulation, whereas the shaded bars represent percent of eyes completely healed when treated with polymer gel (no rHGH). The clear bars represent percent of eyes completely healed when treated with dexamethasone. Each group of animals were treated with daily topical dexamethasone.

### Example 4: Drug Releasing Hydrogel Film

This example describes the preparation of a drug-releasing hydrogel film that is useful for treating conditions of the eye and especially those conditions described herein. The hydrogel is based on thiolated carboxymethyl hyaluronic acid (CMHA-S), supplied by BioTime (Alameda, CA). The hydrogel is formed by crosslinking the CMHA-S with poly(ethylene glycol) diacrylate (PEGDA), also supplied by BioTime. The film is created by drying the hydrogel after incorporating the rHGH.

### Protocol

Tear dry CMHA-S (lyophilized from a pH 6 solution) into small pieces and place in a centrifuge tube. Add 1X PBS buffer (pH 7.4). Vortex tube to mix. Place tube on orbital shaker in 37°C oven. Vortex tube every 15-30 minutes until CMHA-S is fully dissolved. Place PEGDA in a centrifuge tube. Add PBS; vortex to mix. Add desired volume of 100 mg/ml MC (methylcellulose) to CMHA-S solution. Mix gently by inversion. Transfer PEGDA solution to CMHA-S/MC solution. Mix by inversion. Aliquot final solution to prepared wells or mold. Use any remaining final solution in tube to monitor crosslinking. Adequate crosslinking has occurred when the final solution will no longer flow when the tube is inverted, which should occur within 15-30 minutes. Allow the hydrogels to sit at room temperature for a total of 2 hrs (from the time of aliquoting). Transfer hydrogels to an oven (37-45°C), if desired, for 12-24 hrs. Remove dried films from oven; allow films to equilibrate to room temperature for 1-2 hrs. Remove films from wells or mold.

Films were made in 2 ways:
1. In a 5cm x 5cm x 2mm silicone mold (5 mL volume).
2. In 12-well MicroFlexiPerms placed on an acrylic sheet, using enough solution for them to be ∼3mm thick.

For each method, 2 concentrations of CMHA-S were used - either 12 or 16 mg/ml. These films contained PEGDA to have a 1.5:1 thiol:acryl, and also contained 10 mg/ml methylcellulose (MC). The MC provides some flexibility to the films and renders them somewhat mucoadhesive. Disks (6mm diameter) were punched out of films made using method 1, using a standard hole punch. Thus, 4 film types were created, all ∼6mm in diameter - 12mg/ml 2mm; 12 mg/ml 3mm; 16 mg/ml 2mm; 16 mg/ml 3mm.

For initial studies, drug containing films were made with 12 mg/ml CMHA-S, the corresponding amount of PEGDA to provide 1.5:1 thiol:acryl, and 5 mg/ml MC and rHGH at 0.2 mg/ml.

## Claims

1. An ocular drug delivery system, comprising a composition in which a formulation including an active agent that increases insulin growth factor or that alters insulin growth factor binding protein in a subject is dispersed in a pharmaceutical carrier, wherein the active agent is HGH, rHGH, an rHGH mimic, or a combination thereof, wherein the composition is configured for placement in or on an eye of the subject, wherein the composition provides controlled release of an amount of the active agent to the eye effective to promote at least one of ocular surface regeneration, corneal epithelial regeneration, and neural regeneration, and the pharmaceutical carrier is a polymer matrix of a bioerodible polymer comprising a cross- linked hyaluronic acid of a thiolated carboxymethyl hyaluronic acid cross-linked with poly(ethylene glycol) diacrylate.

2. The system of claim 1, wherein the composition is
a) a microparticle suspension, a nanoparticle suspension, a monolithic rod, film, a gel, or a combination thereof, and/ or
b) wherein the composition is formulated as an ocular insert, an implantable depot, a daily topical formulation, a spray formulation, a sustained topical formulation, an injectable fluid, a gel, a film, a sponge , or an ointment.

3. The system of any one of claims 1-2, wherein the pharmaceutical carrier is at least one of a polymer matrix, a topical carrier, an emulsion, gel, film, or an ointment carrier, preferably a polymer matrix.

4. The system of any one of claims 1-3, wherein the formulation further includes
a) a second bioactive agent selected from the group consisting of antibiotics, anti-inflammatory steroids, non-steroidal anti-inflammatory drugs, analgesics, artificial tears solutions, cellular adhesion promoters, growth factors, decongestants, anticholinesterases, antiglaucoma agents, cataract inhibiting drugs, antioxidants, anti-angiogenic drugs, antiallergenics, and combinations thereof; or
b) a second bioactive agent selected from the group consisting of antibiotics, anti-inflammatory steroids, non-steroidal anti-inflammatory drugs, analgesics, artificial tears solutions, cellular adhesion promoters, growth factors, decongestants, anticholinesterases, antiglaucoma agents, cataract inhibiting drugs, antioxidants, anti-angiogenic drugs, antiallergenics, and combinations thereof and wherein the second bioactive agent is an antibiotic selected from the group consisting of ciprofloxacin, gatifloxicin, moxifloxacin, bacitracin, tobramycin, macrolides, polymyxin, gramidicin, erythromycin, tetracycline, and combinations thereof; or wherein the second bioactive agent is an antiinflammatory steroid selected from the group consisting of hydrocortisone, dexamethasone, triamcinolone, prednisolone, fluorometholone, flucinolone acetate, medrysone, and combinations thereof; or wherein the second bioactive agent is a non-steroidal anti-inflammatory drug selected from the group consisting of flurbiprofen sodium, diclofenac sodium, ketorolac, indomethacin, ketoprofen, and combinations thereof; or wherein the second bioactive agent is an analgesic selected from lidocaine, tetracaine, and a combination thereof.

5. A pharmaceutical composition comprising an active agent that increases insulin growth factor, or that alters insulin growth factor binding protein for the use in promoting ocular surface corneal epithelial and neural regeneration resulting in epithelium healing and/or tear film improvement, wherein the active agent is HGH, rHGH, an rHGH mimic, or a combination thereof, preferably rHGH, wherein said pharmaceutical composition comprises a drug delivery composition for administration to, on or around the eye of the subject, the drug delivery composition comprising a formulation including an active agent that increases insulin growth factor or that alters insulin growth factor binding protein, and a polymer matrix provides controlled release of an amount of the active agent to the eye effective to promote ocular surface and corneal epithelial and neural regeneration, wherein the drug delivery system is comprised of a bioerodible polymer comprising a moiety derived from thiolated carboxymethyl hyaluronic acid and a moiety derived from poly(ethylene glycol) diacrylate.

6. The pharmaceutical composition for the use of claim 5, wherein the drug delivery composition is configured for administration to a subconjunctival location, or is configured for injection and or placement into the subconjunctival and or subtenons location; or is configured for administration to an ocular location selected which is the cul de sac, conjunctiva, cornea, subconjunctival limbus, subscleral, intra corneal, periocular region, sub-Tenon's space, or retrobulbar space; or is configured for administration under a contact lens or bandage lens worn in the eye.

7. A drug delivery composition comprising a formulation including an active agent that increases insulin growth factor or that alters insulin growth factor binding protein dispersed in a polymer matrix for the use in preventing or improving delayed ocular wound healing in a subject, wherein the active agent is HGH, rHGH, an rHGH mimic, or a combination thereof, preferably rHGH, wherein the polymer matrix provides controlled release of an amount of the active agent to the eye effective to prevent or improve delayed wound healing, wherein the drug delivery system is comprised of a bioerodible polymer comprising a moiety derived from thiolated carboxymethyl hyaluronic acid and a moiety derived from poly(ethylene glycol) diacrylate.

8. The drug delivery composition for the use of claim 7, for preventing or improving delayed ocular wound healing wherein the subject is expected to have a delayed wound healing and/or decreased innervation condition of the eye, and wherein the delayed wound healing condition is induced, caused by, or associated with treatment of the subject with another drug, wherein preferably the another drug is a steroid, or wherein the another drug includes a member selected from the group consisting of hydrocortisone, dexamethasone, triamcinolone, prednisolone, fluorometholone, flucinolone acetate, medrysone, and combinations thereof.

9. The drug delivery composition for the use of claim 7, wherein the drug delivery system is an ocular insert, solution, film, or ge.

10. A hydrogel film composition for continuous delivery of rHGH to the eye comprising: rHGH in a polymeric matrix comprised of a cross-linked hyaluronic acid for preventing or improving delayed ocular wound healing in a subject, wherein the cross- linked hyaluronic acid is a thiolated carboxymethyl hyaluronic acid cross-linked with poly(ethylene glycol) diacrylate.

11. The composition of claim 10, for preventing or improving delayed ocular wound healing in a subject wherein the delayed ocular wound healing in the subject is caused by or associated with treatment of the subject with another drug, wherein preferably said another drug is a steroid.

12. The composition of any one of claims 10 or 11, wherein the concentration of the rHGH in the matrix is from about 0.05 µg to about 100 µg per milliliter.

13. The composition of any one of claims 10-12, wherein the composition provides continuous release of rHGH for at least 3 days.

## Patentansprüche

1. Okulares Wirkstofffreisetzungssystem, umfassend eine Zusammensetzung, in welcher eine Formulierung beinhaltend einen Wirkstoff, der in einem Subjekt den Insulinwachstumsfaktor erhöht oder das Insulinwachstumsfaktor-Bindungsprotein verändert, in einem pharmazeutischen Träger dispergiert ist, worin der Wirkstoff HGH, rHGH, ein rHGH-Mimetikum oder eine Kombination davon ist, worin die Zusammensetzung für die Platzierung in oder auf einem Auge des Subjekts konfiguriert ist, worin die Zusammensetzung für die kontrollierte Abgabe einer Menge des Wirkstoffs an das Auge sorgt, die zumindest eine von Augenoberflächenregeneration, Hornhautepithelregeneration und Nervenregeneration wirksam fördert, und der pharmazeutische Träger eine Polymermatrix eines bioerodierbaren Polymers umfassend eine vernetzte Hyaluronsäure einer thiolierten Carboxymethyl-Hyaluronsäure, die mit Poly(ethylenglycol)diacrylat vernetzt ist, ist.

2. System nach Anspruch 1, worin die Zusammensetzung Folgendes ist
a) eine Mikropartikelsuspension, eine Nanopartikelsuspension, ein monolithisches Stäbchen, Film, ein Gel oder eine Kombination davon, und/oder
b) worin die Zusammensetzung als ein Okulareinsatz, ein implantierbares Depot, eine tägliche topische Formulierung, eine Sprayformulierung, eine topische Formulierung mit anhaltender Freisetzung, eine injizierbares Fluid, ein Gel, ein Film, ein Schwamm oder eine Salbe formuliert ist.

3. System nach einem der Ansprüche 1-2, worin der pharmazeutische Träger zumindest eines einer Polymermatrix, eines topischen Trägers, einer Emulsion, eines Gels, eines Films oder eines Salbenträgers, vorzugsweise eine Polymermatrix, ist.

4. System nach einem der Ansprüche 1-3, worin die Formulierung ferner Folgendes beinhaltet
a) einen zweiten bioaktiven Wirkstoff, der ausgewählt ist aus der Gruppe bestehend aus Antibiotika, entzündungshemmenden Steroiden, nichtsteroidalen Antiphlogistika, Analgetika, künstlichen Tränenlösungen, Zellanhaftungsvermittlern, Wachstumsfaktoren, abschwellenden Mitteln, Anticholinesterasen, Antiglaukommitteln, Katarakt hemmenden Arzneimitteln, Antioxidanzien, antiangiogenen Arzneimitteln, Antiallergika und Kombinationen davon; oder
b) einen zweiten bioaktiven Wirkstoff, der ausgewählt ist aus der Gruppe bestehend aus Antibiotika, entzündungshemmenden Steroiden, nichtsteroidalen Antiphlogistika, Analgetika, künstlichen Tränenlösungen, Zellanhaftungsvermittlern, Wachstumsfaktoren, abschwellenden Mitteln, Anticholinesterasen, Antiglaukommitteln, Katarakt hemmenden Arzneimitteln, Antioxidanzien, antiangiogenen Arzneimitteln, Antiallergika und Kombinationen davon und worin der zweite bioaktive Wirkstoff ein Antibiotikum ist, das ausgewählt ist aus der Gruppe bestehend aus Ciprofloxacin, Gatifloxicin, Moxifloxacin, Bacitracin, Tobramycin, Makroliden, Polymyxin, Gramidicin, Erythromycin, Tetracyclin und Kombinationen davon; oder worin der zweite bioaktive Wirkstoff ein entzündungshemmendes Steroid ist, das ausgewählt ist aus der Gruppe bestehend aus Hydrocortison, Dexamethason, Triamcinolon, Prednisolon, Fluormetholon, Flucinolonacetat, Medryson und Kombinationen davon; oder worin der zweite bioaktive Wirkstoff ein nichtsteroidales Antiphlogistikum ist, das ausgewählt ist aus der Gruppe bestehend aus Flurbiprofen-Natrium, Diclofenac-Natrium, Ketorolac, Indomethacin, Ketoprofen und Kombinationen davon; oder worin der zweite bioaktive Wirkstoff ein Analgetikum ist, das ausgewählt ist aus Lidocain, Tetracain und einer Kombination davon.

5. Pharmazeutische Zusammensetzung umfassend einen Wirkstoff, der den Insulinwachstumsfaktor erhöht oder der das Insulinwachstumsfaktor-Bindungsprotein verändert, zur Verwendung beim Fördern der Augenoberflächen-, Hornhautepithel- und Nervenregeneration resultierend in Epithelheilung und/oder Tränenfilmverbesserung, worin der Wirkstoff HGH, rHGH, ein rHGH-Mimetikum oder eine Kombination davon, vorzugsweise rHGH, ist, worin besagte pharmazeutische Zusammensetzung eine Wirkstofffreisetzungszusammensetzung zur Verabreichung an, auf oder um das Auge des Subjekts umfasst, wobei die Wirkstofffreisetzungszusammensetzung eine Formulierung beinhaltend einen Wirkstoff umfasst, der den Insulinwachstumsfaktor erhöht oder der das Insulinwachstumsfaktor-Bindungsprotein verändert, und eine Polymermatrix für die kontrollierte Abgabe einer Menge des Wirkstoffs an das Auge sorgt, die die Augenoberflächen- und Hornhautepithel- und Nervenregeneration wirksam fördert, worin das Wirkstofffreisetzungssystem aus einem bioerodierbaren Polymer umfassend eine Moietät, die von thiolierter Carboxymethyl-Hyaluronsäure abgeleitet ist, und eine Moietät, die von Poly(ethylenglycol)diacrylat abgeleitet ist, besteht.

6. Pharmazeutische Zusammensetzung zur Verwendung nach Anspruch 5, worin die Wirkstofffreisetzungszusammensetzung für die Verabreichung an einen subkonjunktivalen Locus konfiguriert ist oder für die Injektion und/oder Platzierung in den subkonjunktivalen und/oder Sub-Tenon-Locus konfiguriert ist; oder für die Verabreichung an einen ausgewählten okularen Locus konfiguriert ist, der die Sackgasse, Bindehaut, Hornhaut, der subkonjunktivale Limbus, die subsklerale, intrakorneale, periokulare Region, der Sub-Tenon-Raum oder retrobulbäre Raum ist; oder für die Verabreichung unter einer Kontaktlinse oder Bandagenlinse, die im Auge getragen wird, konfiguriert ist.

7. Wirkstofffreisetzungszusammensetzung umfassend eine Formulierung beinhaltend einen Wirkstoff, der den Insulinwachstumsfaktor erhöht oder der das Insulinwachstumsfaktor-Bindungsprotein verändert, die in einer Polymermatrix dispergiert ist, zur Verwendung bei der Vorbeugung oder Verbesserung der verzögerten okularen Wundheilung in einem Subjekt, worin der Wirkstoff HGH, rHGH, ein rHGH-Mimetikum oder eine Kombination davon, vorzugsweise rHGH, ist, worin die Polymermatrix für die kontrollierte Abgabe einer Menge des Wirkstoffs an das Auge sorgt, die die verzögerte Wundheilung wirksam verhindert oder verbessert, worin das Wirkstofffreisetzungssystem aus einem bioerodierbaren Polymer umfassend eine Moietät, die von thiolierter Carboxymethyl-Hyaluronsäure abgeleitet ist, und eine Moietät, die von Poly(ethylenglycol)diacrylat abgeleitet ist, besteht.

8. Wirkstofffreisetzungszusammensetzung zur Verwendung nach Anspruch 7, für die Vorbeugung oder Verbesserung einer verzögerten okularen Wundheilung, worin erwartet wird, dass das Subjekt eine verzögerte Wundheilung und/oder einen verminderten Innervationszustand des Auges aufweist und worin der verzögerte Wundheilungszustand mit Behandlung des Subjekts mit einem anderen Arzneimittel induziert wird, dadurch verursacht wird oder damit assoziiert ist, worin, bevorzugt, das andere Arzneimittel ein Steroid ist oder worin das andere Arzneimittel ein Element beinhaltet, das ausgewählt ist aus der Gruppe bestehend aus Hydrocortison, Dexamethason, Triamcinolon, Prednisolon, Fluormetholon, Flucinolonacetat, Medryson und Kombinationen davon.

9. Wirkstofffreisetzungszusammensetzung für die Verwendung nach Anspruch 7, worin das Wirkstofffreisetzungssystem ein Okulareinsatz, Lösung, Film oder Gel ist.

10. Hydrogelfilmzusammensetzung zur kontinuierlichen Freisetzung von rHGH an das Auge, umfassend: rHGH in einer polymeren Matrix bestehend aus einer vernetzten Hyaluronsäure zur Vorbeugung oder Verbesserung einer verzögerten okularen Wundheilung in einem Subjekt, worin die vernetzte Hyaluronsäure eine thiolierte Carboxymethyl-Hyaluronsäure, die mit Poly(ethylenglycol)diacrylat vernetzt ist, ist.

11. Zusammensetzung nach Anspruch 10, zur Vorbeugung oder Verbesserung der verzögerten okularen Wundheilung in einem Subjekt, worin die verzögerte okulare Wundheilung in dem Subjekt bewirkt wird durch die, oder assoziiert ist mit der, Behandlung des Subjekts mit einem anderen Arzneimittel, worin, bevorzugt, besagtes anderes Arzneimittel ein Steroid ist.

12. Zusammensetzung nach einem der Ansprüche 10 oder 11, worin die Konzentration des rHGH in der Matrix von etwa 0,05 µg bis etwa 100 µg pro Milliliter beträgt.

13. Zusammensetzung nach einem der Ansprüche 10-12, worin die Zusammensetzung für die kontinuierliche Abgabe von rHGH für zumindest 3 Tage sorgt.

## Revendications

1. Un système d'apport de médicament oculaire, comprenant une composition dans laquelle une formulation incluant un agent actif qui augmente le facteur de croissance de l'insuline ou qui altère la protéine de liaison du facteur de croissance de l'insuline chez un sujet est dispersée dans un excipient pharmaceutique, dans lequel l'agent actif est un mimétique de HGH, rHGH, un rHGH, ou une combinaison de ces derniers, dans lequel la composition est configurée pour une mise en place dans ou sur un oeil du sujet, dans lequel la composition fournit une libération contrôlée d'une quantité du principe actif à l'oeil, efficace pour promouvoir soit une régénération de la surface oculaire, soit une régénération épithéliale cornéenne, soit une régénération neurale, et l'excipient pharmaceutique est une matrice polymère d'un polymère bioérodable comprenant un acide hyaluronique réticulé d'un acide hyaluronique carboxyméthylique thiolé réticulé avec un poly(éthylène glycol) diacrylate.

2. Le système selon la revendication 1, dans lequel la composition est a) une suspension de microparticules, une suspension de nanoparticules, une tige monolithique, un film, un gel, ou une combinaison de ces derniers, et/ou b) dans lequel la composition est formulée sous la forme d'un implant oculaire, d'un dépôt implantable, d'une formulation topique quotidienne, d'une formulation de pulvérisation, d'une formulation topique prolongée, d'un fluide injectable, d'un gel, d'un film, d'une éponge ou d'un onguent.

3. Le système selon l'une quelconque des revendications 1-2, dans lequel l'excipient pharmaceutique est soit une matrice polymère, soit un excipient topique, soit une émulsion, soit un gel, soit un film, soit un excipient d'onguent, de préférence une matrice polymère.

4. Le système selon l'une quelconque des revendications 1-3, dans lequel la formulation inclut en outre
a) un deuxième agent bioactif sélectionné dans le groupe constitué par des antibiotiques, des stéroïdes anti-inflammatoires, des médicaments anti-inflammatoires non-stéroïdaux, des analgésiques, des solutions de larmes artificielles, des promoteurs d'adhésion cellulaire, des facteurs de croissance, des décongestants, des anticholinestérasiques, des agents antiglaucomateux, des médicaments inhibiteurs de la cataracte, des antioxydants, des médicaments anti-angiogéniques, des antiallergènes, et des combinaisons de ces derniers; ou
b) un deuxième agent bioactif sélectionné dans le groupe constitué par des antibiotiques, des stéroïdes anti-inflammatoires, des médicaments anti-inflammatoires non-stéroïdaux, des analgésiques, des solutions de larmes artificielles, des promoteurs d'adhésion cellulaire, des facteurs de croissance, des décongestants, des anticholinestérasiques, des agents antiglaucomateux, des médicaments inhibiteurs de la cataracte, des antioxydants, des médicaments anti-angiogéniques, des antiallergènes, et des combinaisons de ces derniers et dans lequel le deuxième agent bioactif est un antibiotique sélectionné dans le groupe constitué par la ciprofloxacine, la gatifloxicine, la moxifloxacine, la bacitracine, la tobramycine, les macrolides, la polymyxine, la gramidicine, l'érythromycine, la tétracycline, et des combinaisons de ces derniers; ou dans lequel le deuxième agent bioactif est un stéroïde anti-inflammatoire sélectionné dans le groupe constitué par l'hydrocortisone, la dexaméthasone, la triamcinolone, la prednisolone, la fluorométholone, l'acétate de flucinolone, la médrysone, et des combinaisons de ces derniers; ou dans lequel le deuxième agent bioactif est un médicament anti-inflammatoire non- stéroïdal sélectionné dans le groupe constitué par le flurbiprofène sodique, le diclofénac sodique, le kétorolac, l'indométhacine, le kétoprofène, et des combinaisons de ces derniers; ou dans lequel le deuxième agent bioactif est un analgésique sélectionné parmi la lidocaïne, la tétracaïne, et une combinaison de ces derniers.

5. Une composition pharmaceutique comprenant un agent actif qui augmente le facteur de croissance de l'insuline, ou qui altère la protéine de liaison du facteur de croissance de l'insuline pour l'utilisation dans la promotion de la régénération de la surface oculaire, épithéliale cornéenne et neurale conduisant à la cicatrisation de l'épithélium et/ou à l'amélioration du film de larmes, dans lequel l'agent actif est un mimétique de HGH, rHGH, un rHGH, ou une combinaison de ces derniers, de préférence rHGH, dans lequel ladite composition pharmaceutique comprend une composition d'apport de médicament pour une administration à, sur ou autour de l'oeil du sujet, la composition d'apport de médicament comprenant une formulation incluant un agent actif qui augmente le facteur de croissance de l'insuline ou qui altère la protéine de liaison du facteur de croissance de l'insuline, et une matrice polymère fournit une libération contrôlée d'une quantité de l'agent actif à l'oeil, efficace pour promouvoir la régénération de la surface oculaire et épithéliale cornéenne et neurale, dans laquelle le système d'apport de médicament est constitué d'un polymère bioérodable comprenant une fraction dérivée d'un acide hyaluronique carboxyméthylique thiolé et une fraction dérivée d'un poly(éthylène glycol) diacrylate.

6. La composition pharmaceutique pour l'utilisation selon la revendication 5, dans laquelle la composition d'apport de médicament est configurée pour une administration à un emplacement sous-conjonctival, ou est configurée pour une injection et/ou une mise en place dans l'emplacement sous-conjonctival et/ou la capsule de Tenon; ou est configurée pour une administration à un emplacement oculaire sélectionné qui est le cul de sac, la conjonctive, la cornée, le limbus sous-conjonctival, la région sous-sclérale, intra-cornéenne, périoculaire, la capsule de Tenon ou l'espace rétrobulbaire; ou est configurée pour une administration sous une lentille de contact ou une lentille pansement portée dans l'oeil.

7. Une composition d'apport de médicament comprenant une formulation incluant un agent actif qui augmente le facteur de croissance de l'insuline ou qui altère la protéine de liaison du facteur de croissance de l'insuline, dispersée dans une matrice polymère pour l'utilisation dans la prévention ou l'amélioration d'un retard de cicatrisation des plaies oculaires chez un sujet, dans laquelle l'agent actif est un mimétique de HGH, rHGH, un rHGH, ou une combinaison de ces derniers, de préférence rHGH, dans laquelle la matrice polymère fournit une libération contrôlée d'une quantité de l'agent actif à l'oeil, efficace pour prévenir ou améliorer un retard de cicatrisation des plaies, dans laquelle le système d'apport de médicament est constitué d'un polymère bioérodable comprenant une fraction dérivée d'un acide hyaluronique carboxyméthylique thiolé et une fraction dérivée d'un poly(éthylène glycol) diacrylate.

8. La composition d'apport de médicament pour l'utilisation selon la revendication 7, destinée à prévenir ou améliorer un retard de cicatrisation des plaies oculaires dans laquelle il est attendu que le sujet ait un retard de cicatrisation des plaies et/ou une condition d'innervation diminuée de l'oeil, et dans laquelle la condition de retard de cicatrisation des plaies est induite par, causée par ou associée au traitement du sujet par un autre médicament, dans laquelle de préférence l'autre médicament est un stéroïde, ou dans laquelle l'autre médicament inclut un élément sélectionné dans le groupe constitué par l'hydrocortisone, la dexaméthasone, la triamcinolone, la prednisolone, la fluorométholone, l'acétate de flucinolone, la médrysone, et des combinaisons de ces derniers.

9. La composition d'apport de médicament pour l'utilisation selon la revendication 7, dans laquelle le système d'apport de médicament est un implant oculaire, une solution, un film ou un gel.

10. Une composition de film d'hydrogel pour une administration continue de rHGH à l'oeil, comprenant: rHGH dans une matrice polymère constituée d'un acide hyaluronique réticulé destinée à prévenir ou à améliorer un retard de cicatrisation des plaies oculaires chez un sujet, dans laquelle l'acide hyaluronique réticulé est un acide hyaluronique carboxyméthylique thiolé réticulé avec un poly(éthylène glycol) diacrylate.

11. La composition selon la revendication 10, destinée à prévenir ou améliorer un retard de cicatrisation des plaies oculaires chez un sujet dans laquelle le retard de cicatrisation des plaies oculaires chez le sujet est causé par ou associé à un traitement du sujet par un autre médicament, dans laquelle de préférence ledit autre médicament est un stéroïde.

12. La composition selon l'une quelconque des revendications 10 ou 11, dans laquelle la concentration du rHGH dans la matrice est comprise entre environ 0,05 µg et environ 100 µg par millilitre.

13. La composition selon l'une quelconque des revendications 10-12, dans laquelle la composition fournit une libération continue de rHGH pendant au moins 3 jours.
